# EUROPEAN PATENT SPECIFICATION

(11) **EP 1 356 063 B1**
(45) Date of publication and mention of the grant of the patent: **30.03.2005**
(21) Application number: 01903419.8
(22) Date of filing: 30.01.2001
(51) Int. Cl.: C12N 15/82

(54) **EXPRESSION OF MULTIPLE PROTEINS IN TRANSGENIC PLANTS**
EXPRESSION VON MULTIPLEN PROTEINEN IN TRANSGENEN PFLANZEN
EXPRESSION DE PROTEINES MULTIPLES DANS DES PLANTES TRANSGENIQUES

(43) Date of publication of application: 29.10.2003
(73) Proprietor: Wisconsin Alumini Research Foundation, Madison, WI 53707-7365 (US)
(72) Inventor: VIERSTRA, Richard, D., Madison, WI 53711 (US); WALKER, Joseph, M., Madison, WI 53713 (US)
(74) Representative: Duckworth, Timothy John
(86) International application number: PCT/US2001/002995
(87) International publication number: WO 2002/061100

(56) References cited:
- WO-A-00/11175
- WO-A-95/17514
- WO-A-95/21249
- US-A- 5 162 601
- US-A- 5 773 705
- HONDRED DAVID ET AL: "Use of ubiquitin fusions to augment protein expression in transgenic plants." PLANT PHYSIOLOGY (ROCKVILLE), vol. 119, no. 2, February 1999 (1999-02), pages 713-723, XP002180207 ISSN: 0032-0889
- TAUTZ NORBERT ET AL: "Processing of poly-ubiquitin in the polyprotein of an RNA virus." VIROLOGY, vol. 197, no. 1, 1993, pages 74-85, XP002180208 ISSN: 0042-6822
- BECK VON BODMAN S ET AL: "Expression of multiple eukaryotic genes from a single promoter in Nicotiana" BIO/TECHNOLOGY, NATURE PUBLISHING CO. NEW YORK, US, vol. 13, June 1995 (1995-06), pages 587-591, XP002124728 ISSN: 0733-222X
- CERIANI M FERNANDA ET AL: "Simultaneous accumulation of multiple viral coat proteins from a TEV-NIa based expression vector." PLANT MOLECULAR BIOLOGY, vol. 36, no. 2, 2 January 1998 (1998-01-02), pages 239-248, XP002180209 ISSN: 0167-4412

## Description

In the art of plant genetics, it has now become common practice to create genetically engineered plants, referred to as transgenic plants, which have stably inserted into their chromosomes one or more foreign gene constructions intended to express a novel or foreign protein in the transgenic plants. Techniques exist to insert genes into plant cells and to regenerate whole fertile transgenic plants from such cells. For several important commercial crop species, transgenic seeds are commercially available and are widely planted and harvested.

The most common techniques currently used for creating plant transformation vectors for plant transformations are based on manipulation and construction of the genetic material in bacterial cells followed by the transfer of the genetic materials from the bacterial cells into plant cells. As most commonly practiced, DNA incorporating a protein coding region for the protein of interest is inserted into a plant expression vector which usually includes a promoter and a transcription termination, or polyadenylation sequence, both of which work in plant cells. The combination of a promoter, protein coding sequence, and a polyadenylation sequence is referred to here as a chimeric gene construction or a plant expression cassette. The plant expression vector often also includes a selectable marker gene, or a gene that confers resistance to a chemical selection agent such as an antibiotic or herbicide. Use of such a.selectable marker permits transformed plant cells to be selected from among non-transformed plant cells due to the ability of the transformed plant cells to withstand application of the chemical selection agent to the cells.

Sometimes it is desired that a transgenic plant be constructed that carries more than one foreign gene construction in its genome for more than one gene of interest. If plants are to be altered in their fundamental biochemical characteristics, insertion or alteration of a cascade of enzymes may be needed. While it is possible to incorporate more than one expression cassette into the same plant expression vector, doing so is often not easy or convenient. If two gene cassettes are in the same vector and each includes the same plant promoter or the same polyadenylation sequence, a phenomenon called homologous recombination can occur in the bacterial host which can result in deletion of all of the DNA in the vector which lies in between the two copies of the same promoter. Because there are a limited number of plant promoters known to the art, and since each promoter has different expression characteristics, using two promoters for difference gene cassettes in the same vector can result in different patterns of gene expression for genes that are intended to work in tandem. An alternative approach is to genetically engineer a plant to carry a first inserted gene, called then a transgene, and then to re-engineer the engineered plant to receive a second genetic construction. This approach also has some limitations. First, there are only a few selectable markers known, and one cannot use as a selectable marker in a transformation protocol a marker for which the plant is already resistant. Secondly, when such plant expression cassettes are integrated into the DNA of the plant genome, in general the insertion is at a random location. This gives rise to the so-called "position effect," which is a poorly defined and poorly understood, but widely observed, phenomenon by which the same gene will express at dramatically different levels in different transgenic plants which have the gene inserted in different locations in its genome. Thirdly, the two inserted genes would randomly insert into separate locations in the plant genome, and would thus be genetically unlinked, complicating transfer of the two genes in tandem during plant breeding. Regardless of whether the two inserted genes are introduced at the same time or at two separate times, two genes containing similar sequences in the promoter or polyadenylation signal can adversely affect the activity of each other leading to suppression of both genes, an effect known as co-suppression or gene silencing.

If one wants to engineer a plant to receive a series of enzymes in a cascade intended to produce an end product, one generally wants the enzymes to be produced at relatively similar levels in the cells of the plant. The prior art does not describe convenient methods for achieving such multiple gene expression in plants with comparable or controllable levels of expression among the inserted genes.

Ubiquitin is an abundant protein of 76 amino acids which is present in the cytoplasm and nucleus of all eukaryotic organisms, including plants. Ubiquitin functions as the central component of the ubiquitin-dependent proteolytic pathway, a principle mechanism of amino acid recycling in cells. Ubiquitin is highly conserved and the amino acid sequence of ubiquitin is invariant in all plant species examined so far. Ubiquitin is naturally synthesized as a part of protein fusions which can be polyubiquitins, consisting of multiple ubiquitin monomers in tandem, or ubiquitin extension proteins in which ubiquitin monomers are linked to the amino-terminus of unrelated proteins. The initial fusion proteins produced from such synthesis are naturally processed in vivo to release functional ubiquitin monomers and functional extensions. The enzymes responsible for this activity are known as ubiquitin-specific proteases or UBPs. UBPs are highly specific for ubiquitin and will remove almost any peptide appended by a peptide bond to the carboxyl terminus of ubiquitin, except when proline is the first amino acid of the linked protein at the carboxyl terminus.

Because of its highly stable structure and the natural occurrence of ubiquitin fusions, fusions based on ubiquitin have been previously described as a method to augment the accumulation of unstable or poorly expressed proteins in plants. U. S. Patent No. 5,773,705 describes a system for using a ubiquitin fusion protein strategy for improving the expression of some proteins in plants.

The present invention is summarized in that a transgenic plant includes an artificial genetic construction which includes, 5' to 3', a promoter operable in plants, a protein coding sequence, and a polyadonylation sequence. The protein coding sequence encodes the expression of a chimeric fusion protein which includes the complete amino acid sequence of at least two proteins of interest joined by a ubiquitin linking domain, the ubiquitin linking domain is cleaved under normal physiological conditions in plant cells to release the two proteins of interest.

The present invention is also summarized in a method for making transgenic plants which includes the steps of constructing a plant gene expression cassette including a promoter operable in plants and a polyadenylation sequence operable in plants, the promoter and the polyadenylation sequence operably connected to a protein coding sequence encoding a fusion protein made up of at least two proteins of interest connected by a ubiquitin linking domain, and transforming the plant gene cassette into a plant such that progeny of the plant produces the two proteins of interest in stoichiometric levels.

It is another aspect of the present invention in that a method is described for the expression of multiple proteins in stoichiometric levels in transgenic plants.

Accordingly, the present invention provides a transgenic plant comprising an inserted DNA construction, the DNA construction comprising 5' to 3':
a promoter operable in plant cells;
a coding region encoding a fusion protein, the fusion protein comprising in a
common reading frame:
   a coding region for a first protein of interest;
   a coding region for a plant ubiquitin monomer; and
   a coding region for second protein of interest;
a transcriptional terminator operable in plants,
the coding region effective in the cells of the transgenic plant to produce the fusion protein the cells of the plant, the fusion protein being cleaved by enzymes present in the cells of the plant to release the first and second proteins of interest into the cells of the transgenic plant.

The present invention also provides a transgenic plant comprising an inserted DNA construction, the DNA construction comprising 5' to 3':
a promoter operable in plant cells;
a coding region encoding a fusion protein, the fusion protein comprising in a
common reading frame:
   a region encoding a first protein of interest;
   a region encoding a plant ubiquitin tail region;
   a region encoding an entire plant ubiquitin monomer, and
   a region encoding a second protein of interest;
a transcriptional terminator operable in plants,
the coding region effective in the cells of the transgenic plant to produce the fusion protein in the cells of the plant, the fusion protein being cleaved by enzymes present in the cells of the plant to release the first and second proteins of interest into the cells of the transgenic plant.

The invention also provides the seeds of such plants.

The invention additionally provides an artificial DNA construct comprising 5' to 3':
a promoter operable in plant cells;
a coding region encoding a fusion protein, the fusion protein comprising in a
common reading frame:
   a coding region for a first protein of interest;
   a coding region for a plant ubiquitin monomer; and
   a coding region for second protein of interest;
a transcriptional terminator operable in plants,
the coding region effective with DNA construct is transformed into the cells of a transgenic plant to produce the fusion protein in the cells of the plant, the fusion protein being cleaved by enzymes present in the cells of the plant to release the first and second proteins of interest into the cells of the transgenic plant.

In addition, the invention provides a method for producing at least two proteins in a transgenic plant comprising the steps of
(a) making a DNA construction which includes a promoter operable in plant cells;
   a coding region encoding a fusion protein, the fusion protein comprising in a common reading frame:
   a coding region for a first protein of interest;
   a coding region for a plant ubiquitin monomer; and
   a coding region for second protein of interest;
   a transcriptional terminator operable in plants;
(b) transforming the DNA construct of step (a) into a plant cell and recovering a transgenic plant therefrom; and
(c) cultivating the transgenic plant to maturity such that the fusion protein is produced in the cells of the plant, the fusion protein being cleaved by enzymes in the cells of the plant to release the two proteins of interest into the cells of the transgenic plant.

The invention further provides a method for producing at least two proteins in transgenic plant comprising the steps of:
(a) providing a seed which comprises in its genome an artificial DNA construction which includes:
   a promoter operable in plant cells;
   a coding region encoding a fusion protein, the fusion protein comprising in a
   common reading frame:
      a coding region for a first protein of interest;
      a coding region for a plant ubiquitin monomer; and
      a coding region for second protein of interest;
   a transcriptional terminator operable in plants;
(b) growing the seed of step (a) into a transgenic plant under conditions such that the fusion protein is produced in the cells of the transgenic plant, the fusion protein being cleaved by enzymes in the cells of the transgenic plant to release the two proteins of interest into the cells of the transgenic plant.

Other object advantages and features will become apparent from the following specification when taken in conjunction with the accompanying drawings.

### BRIEF DESCRIPTION OF THE SEVERAL VIEWS OF THE DRAWINGS

Fig. 1 is a schematic representation of the cleavage of a fusion protein constructed in accordance with the present invention.
Fig. 2 is a schematic illustration of several of the chimeric fusion proteins constructed in accordance with the present invention.
Fig. 3 is a graphical representation of some of the results from the example below.

As described here, it is possible to construct a plant expression cassette operable in a transgenic plant, and for that plant expression cassette to encode only a single protein coding sequence, yet still achieve the stoichiometric accumulation of multiple proteins in the transgenic plant. This is accomplished through the strategem of the plant expression cassette including a protein coding sequence which encodes a fusion protein formed of more than one protein of interest, each pair of proteins of interest being joined together in the fusion protein by a linkage domain based on the sequence is expressed in the cells of the host plant, the large fusion protein is expressed. Then the ubiquitin linkage domains of the fusion protein are cleaved by ubiquitin-specific enzymes naturally present in the cells of the plant, this cleavage releasing each of the proteins of interest into the cytosol of the plant cell in approximately equal levels. To facilitate cleavage of the proper proteins in vivo in the plant, it is preferred that the ubiquitin linkage domain include a single ubiquitin head region flanked by two ubiquitin tail regions, one tail at the normal location at the carboxyl terminus of the head region, and another located at the amino terminus of the head region. The second amino terminus tail region facilitates proper release of the upstream protein of interest in a form that retains maximum stability for the upstream protein.

An observation that contributed to the scientific basis for the stratagem used here was the elucidation of the molecular organization of the Bovine Viral Diarrhea Virus (BVDV). The BVDV natively has a rather novel genetic arrangement, in which two viral components are transcribed as a single RNA, which is translated into a fusion protein of two polypeptides joined by a ubiquitin monomer preceded by an additional 14 amino acids from the carboxyl terminus of ubiquitin. The resulting polyprotein is then cleaved in an infected host by indigenous ubiquitin-specific proteases (UBPs) in the host to release the two viral proteins and a free ubiquitin monomer, leaving the 14 amino acid ubiquitin sequence attached to the end of the first protein.

It is disclosed here that the same strategy can be used to make multiple foreign proteins in transgenic plants. In summary, DNA sequences encoding two or more proteins of interest are linked in-frame by a DNA sequence encoding a whole ubiquitin monomer preceded by a DNA sequence encoding the last 14 amino acids of ubiquitin. This fused protein coding sequence is then inserted into a plant expression vector capable of expressing an inserted protein coding sequence in a transgenic plant. The plant expression vector is then transformed into a plant, giving rise to progeny transgenic plants carrying the inserted transgene. In the cells of the progeny transgenic plants, the transgene would be expressed, resulting first in the transcription of an mRNA strand, and then the expression of that strand as a polyprotein or fusion protein. The fusion protein would consist of the two proteins of interest joined by a ubiquitin linkage domain, the ubiquitin linkage domain including at least one ubiquitin monomer and preferably also a 14 amino acid predecessor peptide which corresponds to the carboxyl terminal amino acids of native plant ubiquitin. The fusion protein would be immediately proteolytically cleaved by UBPs present in the cells of the progeny plants, releasing both proteins of interest into the cytoplasm of the cells, and also releasing free ubiquitin monomers. The two proteins, and incidently the free ubiquitin monomers, would be created in exactly the same initial molar quantities. Due to differences in protein stability and degradation, the steady state abundance of the proteins if measured in somatic cells would probably never be precisely equal, but the two proteins would have been produced in precise stoichiometric equality in the cells of the plant. As an incidental side effect, one of the two proteins of interest, the one at the amino terminal end of the fusion protein, would also have at its carboxyl terminus the 14 amino acids from ubiquitin.

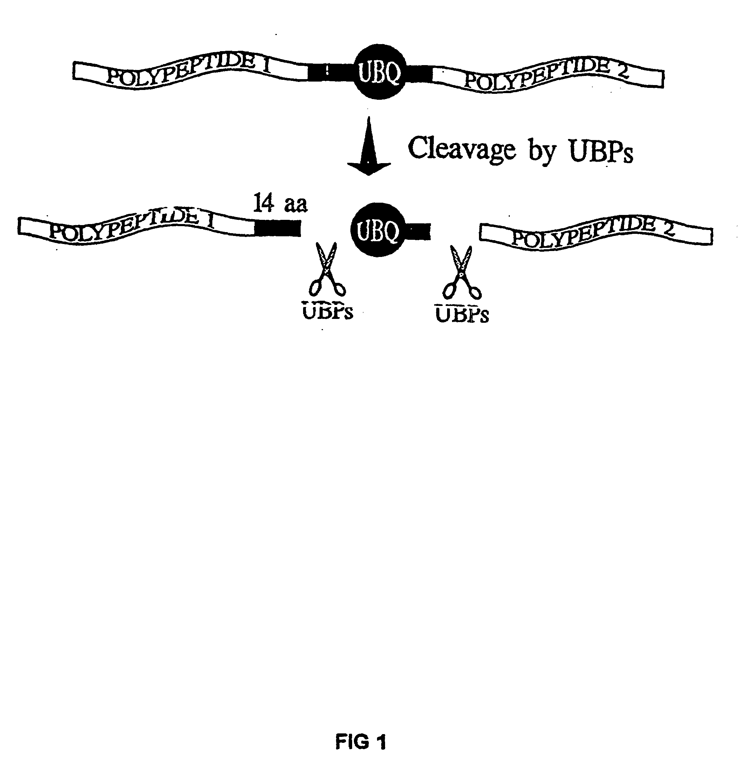

The schematic representation in Fig. 1 illustrates this concept. The fusion protein of polypeptide 1 (the first protein of interest) and polypeptide 2 (the second protein of interest) is interrupted by a linkage region illustrated by a ball labeled UBQ and arms extending in each direction. The ball is the ubiquitin head region and the arm to the right is a ubiquitin tail, so that the combination of the two is a complete ubiquitin monomer. The arm to the left is the upstream additional ubiquitin tail region. The proteolytic cleavage action of the UBP enzymes is represented in the illustration by scissors which cleave the fusion protein immediately upstream of the ubiquitin head region and immediately downstream of the complete ubiquitin monomer. The 14 amino acid upstream ubiquitin tail region remains on the carboxyl terminus of the upstream protein of interest labeled polypeptide 1.

While at its simplest, the concept here is to produce two proteins in roughly equal molar amounts, this concept is not limited to two proteins or to equality. One can simply increase the length of the fusion protein by adding another ubiquitin linkage region and another protein of interest to make a third protein, again in stoichiometric equality with the first two. Another alternative would be that the third protein could be another copy of one of the first two proteins to produce one of the two proteins of interest in quantities approximating twice the levels of the first protein. This logic can be continued indefinitely for the number of proteins and their relative proportions, being limited only by the size of the DNA which can be inserted into the transgenic plant and by the length of the mRNA transcript and protein that can be reliably expressed in the plant of interest.

Since, as far as is known, all plants have exactly the same 76 amino acid ubiquitin sequence, and since all plants absolutely require the presence of UBP enzymes to operate their protein recycling capabilities, the approach described here will work in all higher green plants. While the work described below was performed in tobacco, simply due to the fact that tobacco is a relatively convenient plant to genetically engineer, there is absolutely nothing unique in the biochemistry or genetics of tobacco relevant to this technology as compared with any other higher plant.

It is also envisioned that limited alterations can be made to the native plant ubiquitin protein sequence. Of particular interest, it is specifically envisioned, and may be preferred for some applications, to substitute an arginine residue at amino acid position 48 in the ubiquitin sequence to substitute for the native lysine. This substitution precludes the use of the ubiquitin linkage domain from serving as a site for the formation of a multi-ubiquitin chain. The lysine at residue 48 in native ubiquitin has been shown to be the site for polymerization of additional ubiquitins during formation of such multi-ubiquitin chains which are associated with protein degradation. By the use of the term "ubiquitin" in this application, it is meant to refer to both the native ubiquitin sequence as found in the *AtUBQ11* gene (Callis et al., *Genetics* 139:921-939 (1995), also set forth in SEQ ID NO:2 below), and the same sequence with a lysine at residue 48, as well as other conservative amino acid substitutions which might be made to the native plant sequence resulting in equivalent protein domains. At a nucleotide level, it is preferred that the native plant coding sequence for ubiquitin be used, with or without the substitution for the lysine48, but it is specifically envisioned that other changes to DNA sequence can be made to result in protein coding sequences which would still result in the expression of a protein which functions as a ubiquitin does in plant systems.

While the approach of Agrobacterium-mediated plant transformation is used in the working examples described here, any other plant transformation technique would be equally adapted for use in the present invention. It is now recognized in the art that the method of insertion of an artificial gene into a plant is, for the most part, irrelevant to the later functioning of the gene, referred to as a transgene, in the progeny from that plant. Such transgenes are inherited by progeny through the rules of normal Mendelian inheritance.

The technique of the present invention therefor enables, for the first time, the production of a.plurality of foreign proteins in a transgenic plant, with all the proteins being produced in roughly equivalent amounts. This permits transgenic plants to be created with multiple transgenes in a single transformation event from a non-transgenic variety. And, for those instances in which multiple proteins are sought to be produced in a single transgenic plant, it now becomes possible to produced those proteins in predetermined approximate relationships.

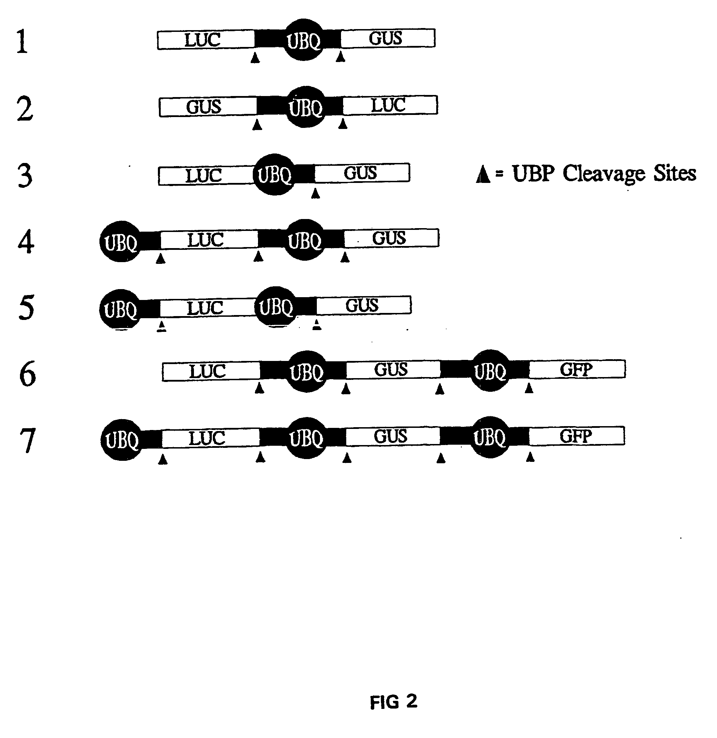

Figure 2 illustrates some of the variations in protein production possible with this technique. Shown in Figure 2 are seven different variations of a multiple protein gene expression cassette, the variations being labeled 1 through 7. The proteins exemplified in these variations are test proteins which create a phenotype that can readily be assayed. The proteins are the beta-glucuronidase gene (GUS), an enzyme which can be detected by colorimetric assay, luciferase, a protein which emits light in an ATP-dependent reaction, and green flourescent protein (GFP), another flourescent protein. In the illustration of Figure 2, the cassettes are transcribed, and the resulting mRNAs are then translated, from left to right. Thus whether one considers either the DNA coding regions or the mRNA, or the resulting fusion protein produced, one protein, or its gene, is always in front of at least one other. The proteins are joined by the ubiquitin linkage domain, illustrated as a ball labeled UBQ, an extension from the ball to the right and, sometimes, an extension from the ball to the left. The ball labeled UBQ represents the ubiquitin head domain and the extensions to the left or to the right represent ubiquitin tail domains.

Thus, the DNA coding sequence for alternative 1 includes, 5' to 3', a coding sequence for the LUC protein, a 14 amino acid ubiquitin tail domain, a ubiquitin head domain, another ubiquitin tail domain, and then the coding region for the GUS protein. When this coding sequence is produced in a transgenic plant, a fusion protein is produced containing the LUC protein connected by the ubiquitin linkage domain to the GUS protein. The fusion protein is then proteolytically cleaved by UBP enzymes in the plant cell to release a complete LUC protein with a 14 amino acid ubiquitin tail domain fused to its carboxyl terminus, a complete GUS protein, and one complete ubiquitin protein monomer.

The other alternatives shown in Figure 2 represent some of the possible variations on this theme. In alternative 2, GUS and LUC coding regions have been reversed. This version will result in the release of the same proteins as did alternative 1, except that there will not be the 14 amino acid ubiquitin tail region appended to the LUC protein. In this case the tail will be on the GUS protein.

In alternative 3, the 14 amino acid ubiquitin tail is omitted from an arrangement otherwise like alternative 1. In this case, neither protein will have the tail appended. While this alternative works, it is thought that the ubiquitin tail region enhances the release of the UBQ from polypeptide 1 by UBPs, and thus alternative 1 is actually preferred over this alternative.

In alternative 4, the arrangement of alternative 1 has been changed to add another complete ubiquitin monomer upstream of the first protein. The addition of this ubiquitin is intended to aid in expression of the entire fusion protein in the plant cells. Since ubiquitin is ubiquitous and abundantly produced in plant cells, it has come to be understood that the ubiquitin protein is one that the protein synthesis machinery of plants can produce with some efficiency. Thus adding the ubiquitin monomer at the amino terminus of the protein can improve the level of production of the fusion protein itself, thus boosting the expression level of all of the proteins in the fusion.

Alternative 5 is similar to alternative 4 except that the ubiquitin tail domain upstream of the ubiquitin domain in the interior of the coding region has been omitted.

Alternatives 6 and 7 are intended to illustrate three protein fusions of LUC, GUS and GFP. In each of these arrangements, there are two linkage domains, each with a complete ubiquitin monomer preceded by an extra tail domain. Alternative 7 adds a complete ubiquitin monomer at the amino terminus of the fusion protein, a feature not included in Alternative 6.

### EXAMPLES

### Construction of LUC-U3'-UBQ-GUS Fusion

A DNA.coding sequence for a fusion of luciferase, a ubiquitin linkage domain and beta-glucuronidase, as illustrated in Alternative 1 of Figure 2, was constructed using standard cloning techniques using PCR oligonucleotide primers to generate the protein fusion junctions. To assemble an expression cassette operable in plants, the following components were joined together in order 5' to 3': a PCR fragment containing the CaMV 35S promoter (Barton *er* al. *Plant Physiol*. 85:1103-1109 (1987)), the 39-bp of the 5' upstream untranslated region from alfalfa mosaic virus (Gehrke et al. *Biochemistry*, 22:5157-5164, 1983), the full length coding region for the luciferase protein from pAB14016LBS (de Wet et al. *Mol. Cell Biol*. 7:725-737 (1983)), followed in frame by the last 42-bp of the third repeat of the *AtUBQ11* gene (Callis et al. *Genetics,* 139:921-939, 1995), followed in frame by the first 10-bp of the same ubiquitin coding region. All these elements were generated from a luciferase expression vector using the standard M13-20 primer: and the antisense primer:

The luciferase coding region in this vector had been previously modified to contain a translationally silent KasI site 39-bp downstream from the start codon. The ubiquitin coding sequence had been previously modified to contain a translationally silent BglII site 10-bp downstream from its start codon and a translationally silent SacII site 5-bp upstream from the carboxy terminal glycine. The resulting PCR fragment was cloned into an EcoRV digest of the vector pBluescriptII KS (Stratagene) and then excised as a KpnI/BglII fragment. The KpnI/BglII fragment thus produced, CaMV35S-AMV-LUC-U3'-UBQ, was cloned into a KpnI/BglII digest of an expression vector containing the same CaMV35S-AMV cassette followed by the third repeat of the *AtUBQ11* coding region translationally fused to the GUS-coding region and the nopaline synthase transcriptional terminator from the plasmid pCMC1100 (McCabe et al. *Biotechnology* 6:923-926 (1988)). The GUS-coding region had been previously modified to contain a translationally silent XhoI site 31-bp downstream from the start codon. The completed expression cassette for the fusion protein was then cloned into the binary vector BIN19 (Clontech) as a KpnI/SalI fragment.

The entire sequence of the expression cassette for this construction is set forth in SEQ ID NO:1 below. In SEQ ID NO:1, base pairs numbered 1 to 452 are the CaMV35S promoter beginning at the KpnI site. Following that are the 39 base pairs of the AMV untranslated leader sequence. The protein coding region for the fusion protein begins at base pair 490 and continues on to 4216. Bases 490 through 2140 contain the coding sequence for the luciferase gene (amino acids 1 through 550 in the protein). The next 42 bases (2141 to 2182) are the coding region for a ubiquitin tail region (amino acids 551 through 564). Then follows the coding region for a complete ubiquitin monomer from bases 2183 through 2410, amino acids 565 through 640. The coding region for the second protein of interest, in this case GUS, begins at base 2411 and continues on through base 4216, amino acids 641 through 1242. The remaining bases in the sequence, bases 4217 to 4544, are a nopaline synthase polyadenylation sequence ending in a SalI site.

### Plant Transformations

The completed BIN19 plasmid was then introduced directly into *Agrobacterium tumefaciens* strain LBA4404, which was then used to transform tobacco (*Nicotiana tabacum* cv Xanthi) leaf disks using the method of Cherry et al. *Plant Cell* 5:565-575 (1993). Cells in culture were selected for kanamycin resistance and stably transformed kanamycin resistant plants were recovered. Transgenic plants were transferred to soil and grown to maturity in a greenhouse.

### Enzyme Extractions and Analysis

In order to evaluate the enzyme levels in the cells of the plants, total proteins were extracted from the youngest expanded leaves of transformed plants. The total soluble protein in the samples was measured by the Bradford method (Bradford et al. *Anal. Biochem*. 72:248-254 (1976)). Total soluble protein was extracted by grinding transgenic plant tissue in 25 mM Tris-phosphate (pH7.8), 20 mM sodium metabisulfite, 2 mM 1,2-diaminecyclohexane-N,N,N',N'-tetraacetic acid, 10% (v/v) glycerol, and 1% (v/v) Triton X-100. For GUS activity measurement, a fluorometric assay was used with 4-methylumbelliferyl β-D-glucuronide as the substrate. LUC activity was determined using a chemiluminescence assay system from Promega Corporation.

### Immunoblot Analysis

Proteins were separated by SDS-PAGE, electro-transferred to immobilon-P membrane (Millipore), and then subjected to immunoblot assay as previously described in van Nocker et al. *J. Biol. Chem*. 268:24766-24773 (1993). Rabbit polyclonal antibodies were prepared against partially purified GUS protein purchased from Sigma which was further purified by Mono-Q ion-exchange chromatography (Pharmacia) before injection into a New Zealand White rabbit as a one-to-one mixture of Freund's adjuvant and antigen following a standard course of intradermal injections. LUC antibodies were purchased from Promega Corporation. The immunoreactive proteins were identified with alkaline phosphatase-coupled IgGs (Kirkegaard and Perry Laboratories).

### Results

Transgenic tobacco plants have been recovered transformed with a transgene expressing a fusion protein of LUC and GUS with the ubiquitin linkage domain placed between them (Alternative 1 of Fig. 2). Analysis of the resulting proteins confirmed that free forms of the proteins were detected for both proteins in the tissues of the plants.

This experiment has been repeated in Arabidopsis and again both proteins were produced and detected in free form.

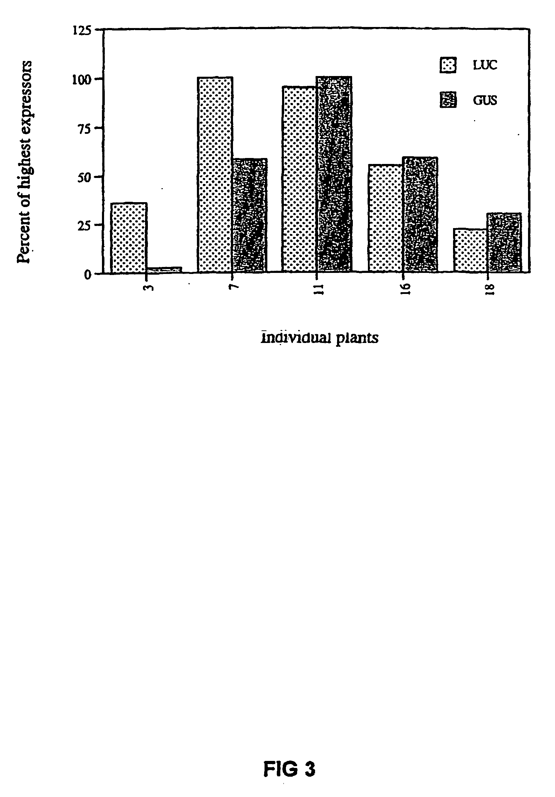

Transformation of the vector alternative 1 into tobacco plants resulted in plants expressing both luciferase and beta-glucuronidase. Analysis by SDS-PAGE and western blotting showed proteins corresponding to the full length beta-glucuronidase and the luciferase protein with the added 14 amino acid luciferase tail. Quantitative assays on five independently transformed plants revealed levels of the two reporter enzymes which are similar. The results are presented in the form of bar graphs in Fig. 3.

## Claims

1. A transgenic plant comprising an inserted DNA construction, the DNA construction comprising 5' to 3':
a promoter operable in plant cells;
a coding region encoding a fusion protein, the fusion protein comprising in a common reading frame
a coding region for a first protein of interest;
a coding region for a plant ubiquitin monomer; and
a coding region for second protein of interest;
a transcriptional terminator operable in plants,
the coding region effective in the cells of the transgenic plant to produce the fusion protein in the cells of the plant, the fusion protein being cleaved by enzymes present in the cells of the plant to release the first and second proteins of interest into the cells of the transgenic plant.

2. Seeds of the plant of claim 1.

3. The plant as claimed in claim 1 where in the coding sequence for the plant ubiquitin monomer encodes an arginine at amino acid position 48 in native plant ubiquitin sequence.

4. A transgenic plant comprising an inserted DNA construction, the DNA construction comprising 5' to 3' :
a promoter operable in plant cells;
a coding region encoding a fusion protein, the fusion protein comprising in a common reading frame
a region encoding a first protein of interest;
a region encoding a plant ubiquitin tail region;
a region encoding an entire plant ubiquitin monomer; and
a region encoding a second protein of interest;
a transcriptional terminator operable in plants,
the coding region effective in the cells of the transgenic plant to produce the fusion protein in the cells of the plant, the fusion protein being cleaved by enzymes present in the cells of the plant to release the first and second proteins of interest into the cells of the transgenic plant.

5. Seeds of the plant of claim 4.

6. The plant as claimed in claim 4 where in the region encoding the plant ubiquitin monomer encodes an arginine at amino acid position 48 in native plant ubiquitin sequence.

7. An artificial DNA construct comprising 5' to 3':
a promoter operable in plant cells;
a coding region encoding a fusion protein, the fusion protein comprising in a common reading frame
a coding region for a first protein of interest;
a coding region for a plant ubiquitin monomer; and
a coding region for second protein of interest;
a transcriptional terminator operable in plants,
the coding region effective when the DNA construct is transformed into the cells of a transgenic plant to produce the fusion protein in the cells of the plant, the fusion protein being cleaved by enzymes present in the cells of the plant to release the first and second proteins of interest into the cells of the transgenic plant.

8. The DNA construct of claim 7 wherein the fusion protein also includes at the end of the first protein of interest a coding region encoding a tail domain from plant ubiquitin.

9. The DNA construct of claim 7 wherein the coding region for ubiquitin includes a substitution of a codon for the amino acid arginine for the codon for the amino acid lysine present at position 48 in the native ubiquitin sequence.

10. A method for producing at least two proteins in a transgenic plant comprising the steps of
(a) making a DNA construction which includes a promoter operable in plant cells;
a coding region encoding a fusion protein, the fusion protein comprising in a common reading frame
a coding region for a first protein of interest;
a coding region for a plant ubiquitin monomer; and
a coding region for second protein of interest;
a transcriptional terminator operable in plants;
(b) transforming the DNA construction of step (a) into a plant cell and recovering a transgenic plant therefrom; and
(c) cultivating the transgenic plant to maturity such that the fusion protein is produced in the cells of the plant, the fusion protein being cleaved by enzymes in the cells of the plant to release the two proteins of interest into the cells of the transgenic plant.

11. The method of claim 10 wherein the fusion protein also includes a ubiquitin tail domain at the end of the first protein of interest.

12. The method of claim 10 wherein the coding region for the plant ubiquitin includes an arginine residue at amino acid position 48.

13. A method for producing at least two proteins in transgenic plant comprising the steps of
(a) providing a seed which comprises in its genome an artificial DNA construction which includes
a promoter operable in plant cells;
a coding region encoding a fusion protein, the fusion protein comprising in a common reading frame
a coding region for a first protein of interest;
a coding region for a plant ubiquitin monomer; and
a coding region for second protein of interest;
a transcriptional terminator operable in plants;
(b) growing the seed of step (a) into a transgenic plant under conditions such that the fusion protein is produced in the cells of the transgenic plant, the fusion protein being cleaved by enzymes in the cells of the transgenic plant to release the two proteins of interest into the cells of the transgenic plant.

14. The method of claim 13 wherein the fusion protein also includes a ubiquitin tail domain at the end of the first protein of interest.

15. The method of claim 13 wherein the coding region for the plant ubiquitin includes an arginine residue at amino acid position 48.

## Patentansprüche

1. Transgene Pflanze, umfassend ein insertiertes DNA-Konstrukt, wobei das DNA-Konstrukt von 5' nach 3' folgendes umfasst:
in Pflanzenzellen operativer Promotor;
Kodierungsbereich, der ein Fusionsprotein kodiert, wobei das Fusionsprotein in einem gemeinsamen Leserahmen folgendes umfasst:
Kodierungsbereich für ein erstes Protein von Interesse;
Kodierungsbereich für ein Pflanzen-Ubiquitin-Monomer; und
Kodierungsbereich für ein zweites Protein von Interesse;
in Pflanzen operativer Transkriptionsterminator, wobei der Kodierungsbereich in den Zellen der transgenen Pftanze wirksam ist, um das Fusionsprotein in den Zellen der Pflanze herzustellen, wobei das Fusionsprotein durch Enzyme, die in den Zellen der Pflanze vorhanden sind, gespalten wird, um die ersten und zweiten Proteine von Interesse in die Zellen der transgenen Pflanze freizusetzen.

2. Samen der Pflanze gemäß Anspruch 1.

3. Pflanze gemäß Anspruch 1, wobei die Kodierungssequenz für das Pflanzen-Ubiquitin-Monomer ein Arginin an der Aminosäureposition 48 der nativen Pflanzen-Ubiquitin-Sequenz kodiert.

4. Transgene Pflanze, umfassend ein insertiertes DNA-Konstrukt, wobei das DNA-Konstrukt von 5' nach 3' folgendes umfasst:
in Pflanzenzellen operativer Promotor;
Kodierungsbereich, der ein Fusionsprotein kodiert, wobei das Fusionsprotein in einem gemeinsamen Leserahmen folgendes umfasst:
Kodierungsbereich, der ein erstes Protein von Interesse kodiert;
Kodierungsbereich, der einen Schwanzbereich von Pflanzen-Ubiquitin kodiert;
Kodierungsbereich, der ein vollständiges Pflanzen-Ubiquitin-Monomer kodiert; und
Kodierungsbereich, der ein zweites Protein von Interesse kodiert;
in Pflanzen operativer Transkriptionsterminator, wobei der Kodierungsbereich in den Zellen der transgenen Pflanze wirksam ist, um das Fusionsprotein in den Zellen der Pflanze herzustellen, wobei das Fusionsprotein durch Enzyme, die in den Zellen der Pflanze vorhanden sind, gespalten wird, um die ersten und zweiten Proteine von Interesse in die Zellen der transgenen Pflanze freizusetzen.

5. Samen der Pflanze gemäß Anspruch 4.

6. Pflanze gemäß Anspruch 4, wobei der Kodierungsbereich für das Pflanzen-Ubiquitin-Monomer ein Arginin an der Aminosäureposition 48 der nativen Pflanzen-Ubiquitin-Sequenz kodiert.

7. Künstliches DNA-Konstrukt, das von 5' nach 3' folgendes umfasst:
in Pflanzenzellen operativer Promotor,
Kodierungsbereich, der ein Fusionsprotein kodiert, wobei das
Fusionsprotein in einem gemeinsamen Leserahmen folgendes umfasst:
Kodierungsbereich für ein erstes Protein von Interesse;
Kodierungsbereich für ein Pflanzen-Ubiquitin-Monomer; und
Kodierungsbereich für ein zweites Protein von Interesse;
in Pflanzen operativer Transkriptionsterminator, wobei der Kodierungsbereich wirksam ist, wenn das DNA-Konstrukt in die Zellen einer transgenen Pflanze transformiert ist, um das Fusionsprotein in den Zellen der Pflanze herzustellen, wobei das Fusionsprotein durch Enzyme, die in den Zellen der Pflanze vorhanden sind, gespalten wird, um die ersten und die zweiten Proteine von Interesse in die Zellen der transgenen Pflanze freizusetzen.

8. DNA-Konstrukt gemäß Anspruch 7, wobei das Fusionsprotein am Ende des ersten Proteins von Interesse auch einen Kodierungsbereich, der eine Schwanzdomäne von Pflanzen-Ubiquitin kodiert, umfasst.

9. DNA-Konstrukt gemäß Anspruch 7, wobei der Kodierungsbereich für Ubiquitin eine Substitution eines Kodons für die Aminosäure Arginin für das Kodon für die Aminosäure Lysin, die an der Position 48 der nativen Ubiquitin-Sequenz vorhanden ist, umfasst.

10. Verfahren zum Herstellen von wenigstens zwei Proteinen in einer transgenen Pflanze, umfassend folgende Stufen:
(a) Herstellen eines DNA-Kontrukts, das folgendes umfasst:
in Pflanzenzellen operativer Promotor;
Kodierungsbereich, der ein Fusionsprotein kodiert, wobei das
Fusionsprotein in einem gemeinsamen Leserahmen folgendes umfasst:
Kodierungsbereich für ein erstes Protein von Interesse;
Kodierungsbereich für ein Pflanzen-Ubiquitin-Monomer; und
Kodierungsbereich für ein zweites Protein von Interesse;
in Pflanzen operativer Transkriptionsterminator;
(b) Transformieren des DNA-Kontrukts aus Schritt (a) in eine Pflanzenzelle und Gewinnen einer transgenen Pftanze davon; und
(c) Kultivieren der transgenen Pflanze bis zur Reife, so dass das Fusionsprotein in den Zellen der Pflanze hergestellt wird, wobei das Fusionsprotein durch Enzyme in den Zellen der Pflanze gespalten wird, um die beiden Proteine von Interesse in die Zellen der transgenen Pflanze freizusetzen.

11. Verfahren gemäß Anspruch 10, wobei das Fusionsprotein am Ende des ersten Proteins von Interesse auch eine Ubiquitin-Schwanzdomäne umfasst.

12. Verfahren gemäß Anspruch 10, wobei der Kodierungsbereich für das Pflanzen-Ubiquitin einen Arginin-Rest an der Aminosäureposition 48 umfasst.

13. Verfahren zum Herstellen von wenigstens zwei Proteinen in einer transgenen Pflanze, umfassend folgende Stufen:
(a) Bereitstellen eines Samens, der in seinem Genom ein künstliches DNA-Konstrukt umfasst, umfassend folgendes:
in Pflanzenzellen operativer Promotor;
Kodierungsbereich, der ein Fusionsprotein kodiert, wobei das
Fusionsprotein in einem gemeinsamen Leserahmen folgendes umfasst:
Kodierungsbereich für ein erstes Protein von Interesse;
Kodierungsbereich für ein Pflanzen-Ubiquitin-Monomer; und
Kodierungsbereich für ein zweites Protein von Interesse;
in Pflanzen operativer Transkriptionsterminator;
(b) Heranwachsen des Samens aus Schritt (a) in eine transgene Pflanze unter solchen Bedingungen, dass das Fusionsprotein in den Zellen der transgenen Pflanze hergestellt wird, wobei das Fusionsprotein durch Enzyme in den Zellen der Pflanze gespalten wird, um die beiden Proteine von Interesse in die Zellen der transgenen Pflanze freizusetzen.

14. Verfahren gemäß Anspruch 13, wobei das Fusionsprotein am Ende des ersten Proteins von Interesse auch eine Ubiquitin-Schwanzdomäne umfasst.

15. Verfahren gemäß Anspruch 13, wobei der Kodierungsbereich für das Pflanzen-Ubiquitin einen Arginin-Rest an der Aminosäureposition 48 umfasst.

## Revendications

1. plante transgénique dans laquelle est insérée une construction d'ADN qui comporte, de 5' à 3' :
- un promoteur qui peut fonctionner dans les cellules végétales,
- une région codante qui code une protéine de fusion, laquelle région codante comporte, dans un cadre de lecture commun:
une région codant une première protéine d'intérêt,
une région codant un monomère d'ubiquitine végétale,
et une région codant une deuxième protéine d'intérêt,
- et un terminateur de transcription qui peut fonctionner chez les végétaux,
ladite région codante fonctionnant efficacement dans les cellules de la planté transgénique afin que la protéine de fusion soit produite dans les cellules de cette plante, laquelle protéine de fusion est ensuite clivée par des enzymes présentes dans les cellules de la plante pour libérer la première protéine d'intérêt et la deuxième protéine d'intérêt dans les cellules de la plante transgénique.

2. Semences d'une plante conforme à la revendication 1.

3. Plante conforme à la revendication 1, dans laquelle la séquence codant le monomère d'ubiquitine végétale code un résidu arginine en position 48 de la séquence d'acides aminés de l'ubiquitine végétale naturelle.

4. Plante transgénique dans laquelle est insérée une construction d'ADN qui comporte, de 5' à 3' :
- un promoteur qui peut fonctionner dans les cellules végétales,
- une région codante qui code une protéine de fusion, laquelle région codante comporte, dans un cadre de lecture commun :
une région codant une première protéine d'intérêt,
une région codant un domaine de queue d'ubiquitine végétale,
une région codant un monomère complet d'ubiquitine végétale,
et une région codant une deuxième protéine d'intérêt,
- et un terminateur de transcription qui peut fonctionner chez les végétaux,
ladite région codante fonctionnant efficacement dans les cellules de la plante transgénique afin que la protéine de fusion soit produite dans les cellules de cette plante, laquelle protéine de fusion est ensuite clivée par des enzymes présentes dans les cellules de la plante pour libérer la première protéine d'intérêt et la deuxième protéine d'intérêt dans les cellules de la plante transgénique.

5. Semences d'une plante conforme à la revendication 4.

6. Plante conforme à la revendication 4, dans laquelle la séquence codant le monomère d'ubiquitine végétale code un résidu arginine en position 48 de la séquence d'acides aminés de l'ubiquitine végétale naturelle.

7. Construction d'ADN artificielle, qui comporte, de 5' à 3'
- un promoteur qui peut fonctionner dans les cellules végétales,
- une région codante qui code une protéine de fusion, laquelle région codante comporte, dans un cadre de lecture commun :
une région codant une première protéine d'intérêt,
une région codant un monomère d'ubiquitine végétale,
et une région codant une deuxième protéine d'intérêt,
- et un terminateur de transcription qui peut fonctionner chez les végétaux,
ladite région codante fonctionnant efficacement, quand la construction d'ADN est introduite dans les cellules d'une plante pour transformer celle-ci et la rendre transgénique, afin que la protéine de fusion soit produite dans les cellules de cette plante, laquelle protéine de fusion est ensuite clivée par des enzymes présentes dans les cellules de la plante pour libérer la première protéine d'intérêt et la deuxième protéine d'intérêt dans les cellules de la plante transgénique.

8. Construction d'ADN conformé à la revendication 7, dans laquelle la région codant la protéine de fusion comporte en outre, à la fin de la région codant la première protéine d'intérêt, une région codant un domaine de queue d'ubiquitine végétale.

9. Construction d'ADN conforme à la revendication 7, dans laquelle, dans la région codant l'ubiquitine, le codon du résidu lysine présent en position 48 de la séquence d'acides aminés de l'ubiquitine naturelle est remplacé par un codon de résidu arginine.

10. Procédé permettant de produire au moins deux protéines dans une plante transgénique, lequel procédé comporte les étapes suivantes :
a) préparer une construction d'ADN comportant :
- un promoteur qui peut fonctionner dans les cellules végétales,
- une région codante qui code une protéine de fusion, laquelle région codante comporte, dans un cadre de lecture commun :
une région codant une première protéine d'intérêt,
une région codant un monomère d'ubiquitine végétale,
et une région codant une deuxième protéine d'intérêt,
- et un terminateur de transcription qui peut fonctionner chez les végétaux ;
b) introduire la construction d'ADN de l'étape (a) dans une cellule végétale pour transformer celle-ci, et obtenir à partir de cette cellule une plante transgénique,
c) et cultiver la plante transgénique jusqu'à maturité, de telle façon que la protéine de fusion soit produite dans les cellules de cette plante, laquelle protéine de fusion est ensuite clivée par des enzymes présentes dans les cellules de la plante pour libérer les deux protéines d'intérêt dans les cellules de la plante transgénique.

11. Procédé conforme à la revendication 10, dans lequel la protéine de fusion comporte en outre, à la fin de la première protéine d'intérêt, un domaine de queue d'ubiquitine.

12. Procédé conforme à la revendication 10, dans lequel la région codant l'ubiquitine végétale comporte un codon donnant un résidu arginine en position 48 de la séquence d'acides aminés.

13. Procédé permettant de produire au moins deux protéines dans une plante transgénique, lequel procédé comporte les étapes suivantes:
a) préparer une semence qui contient, dans son génome, une construction d'ADN artificielle comportant :
- un promoteur qui peut fonctionner dans les cellules végétales,
- une région codante qui code une protéine de fusion, laquelle région codante comporte, dans un cadre de lecture commun :
une région codant une première protéine d'intérêt,
une région codant un monomère d'ubiquitine végétale,
et une région codant une deuxième protéine d'intérêt,
- et un terminateur de transcription qui peut fonctionner chez les végétaux ;
b) et faire pousser la semence de l'étape (a) pour qu'elle donne une plante transgénique, dans des conditions telles que la protéine de fusion soit produite dans les cellules de cette plante, laquelle protéine de fusion est ensuite clivée par des enzymes présentes dans les cellules de la plante transgénique pour libérer les deux protéines d'intérêt dans les cellules de la plante transgénique.

14. Procédé conforme à la revendication 13, dans lequel la protéine de fusion comporte en outre, à la fin de la première protéine d'intérêt, un domaine de queue d'ubiquitine.

15. Procédé conforme à la revendication 13, dans lequel la région codant l'ubiquitine végétale comporte un codon donnant un résidu arginine en position 48 de la séquence d'acides aminés.


### SEQUENCE LISTING

<110> Vierstra, Richard D Walker, Joseph M
<120> Production of Multiple Proteins in Plants
<130> 960296.96501
<140>
   <141>
<160> 2
<170> PatentIn Ver. 2.1
<210> 1
   <211> 4544
   <212> DNA
   <213> Artificial Sequence
<220>
   <221> CDS
   <222> (491)..(4216)
<220>
   <223> Description of Artificial Sequence:Fusion Protein Expression Cassette
<400> 1
   ggtaccgggc cccggccggc tagcgtcgag gaacatggtg gagcacgaca ctctcgtcta 60
   ctccaagaat atcaaagata cagtctcaga agaccaaagg gctattgaga cttttcaaca 120
   aagggtaata tcgggaaacc tcctcggatt ccattgccca gctatctgtc acttcatcaa 180
   aaggacagta gaaaaggaag gtggcaccta caaatgccat cattgcgata aaggaaaggc 240
   tatcgttcaa gatgcctctg ccgacagtgg tcccaaagat ggacccccac ccacgaggag 300
   catcgtggaa aaagaagacg ttccaaccac gtcttcaaag caagtggatt gatgtgatat 360
   ctccactgac gtaagggatg acgcacaatc ccactatcct tcgcaagacc cttcctctat 420
   ataaggaagt tcatttcatt tggagaggac caagcttttt atttttaatt ttctttcaaa 480
   tacttccacc atg gaa gac gcc aaa aac ata aag aaa ggc ccg gcg cca 529
   Met Glu Asp Ala Lys Asn Ile Lys Lys Gly Pro Ala Pro
   1 5 10
   ttc tat cct cta gag gat gga acc gct gga gag caa ctg cat aag gct 577
   Phe Tyr Pro Leu Glu Asp Gly Thr Ala Gly Glu Gln Leu His Lys Ala
   15 20 25
   atg aag aga tac gcc ctg gtt cct gga aca att gct ttt aca gat gca 625
   Met Lys Arg Tyr Ala Leu Val Pro Gly Thr Ile Ala Phe Thr Asp Ala
   30 35 40 45
   cat atc gag gtg aac atc acg tac gcg gaa tac ttc gaa atg tcc gtt 673
   His Ile Glu Val Asn Ile Thr Tyr Ala Glu Tyr Phe Glu Met Ser Val
   50 55 60
   cgg ttg gca gaa gct atg aaa cga tat ggg ctg aat aca aat cac aga 721
   Arg Leu Ala Glu Ala Met Lys Arg Tyr Gly Leu Asn Thr Asn His Arg
   65 70 75
   atc gtc gta tgc agt gaa aac tct ctt caa ttc ttt atg ccg gtg ttg     769
   Ile Val Val Cys Ser Glu Asn Ser Leu Gln Phe Phe Met Pro Val Leu
           80                  85                  90
   ggc gcg tta ttt atc gga gtt gca gtt gcg ccc gcg aac gac att tat     817
   Gly Ala Leu Phe Ile Gly Val Ala Val Ala Pro Ala Asn Asp Ile Tyr
       95                  100                 105
   aat gaa cgt gaa ttg ctc aac agt atg aac att tcg cag cct acc gta     865
   Asn Glu Arg Glu Leu Leu Asn Ser Met Asn Ile Ser Gln Pro Thr Val
   110                 115                 120                 125
   gtg ttt gtt tcc aaa aag ggg ttg caa aaa att ttg aac gtg caa aaa     913
   Val Phe Val Ser Lys Lys Gly Leu Gln Lys Ile Leu Asn Val Gln Lys
                   130                 135                 140
   aaa tta cca ata atc cag aaa att att atc atg gat tct aaa acg gat     961
   Lys Leu Pro Ile Ile Gln Lys Ile Ile Ile Met Asp Ser Lys Thr Asp
               145                 150                 155
   tac cag gga ttt cag tcg atg tac acg ttc gtc aca tct cat cta cct     1009
   Tyr Gln Gly Phe Gln Ser Met Tyr Thr Phe Val Thr Ser His Leu Pro
           160                 165                 170
   ccc ggt ttt aat gaa tac gat ttt gta cca gag tcc ttt gat cgt gac     1057
   Pro Gly Phe Asn Glu Tyr Asp Phe Val Pro Glu Ser Phe Asp Arg Asp
       175                 180                 185
   aaa aca att gca ctg ata atg aat tcc tct gga tct act ggg tta cct     1105
   Lys Thr Ile Ala Leu Ile Met Asn Ser Ser Gly Ser Thr Gly Leu Pro
   190                 195                 200                 205
   aag ggt gtg gcc ctt ccg cat aga act gcc tgc gtc aga ttc tcg cat     1153
   Lys Gly Val Ala Leu Pro His Arg Thr Ala Cys Val Arg Phe Ser His
                   210                 215                 220
   gcc aga gat cct att ttt ggc aat caa atc att ccg gat act gcg att     1201
   Ala Arg Asp Pro Ile Phe Gly Asn Gln Ile Ile Pro Asp Thr Ala Ile
               225                 230                 235
   tta agt gtt gtt cca ttc cat cac ggt ttt gga atg ttt act aca ctc     1249
   Leu Ser Val Val Pro Phe His His Gly Phe Gly Met Phe Thr Thr Leu
           240                 245                 250
   gga tat ttg ata tgt gga ttt cga gtc gtc tta atg tat aga ttt gaa     1297
   Gly Tyr Leu Ile Cys Gly Phe Arg Val Val Leu Met Tyr Arg Phe Glu
       255                 260                 265
   gaa gag ctg ttt tta cga tcc ctt cag gat tac aaa att caa agt gcg     1345
   Glu Glu Leu Phe Leu Arg Ser Leu Gln Asp Tyr Lys Ile Gln Ser Ala
   270                 275                 280                 285
   ttg cta gta cca acc cta ttt tca ttc ttc gcc aaa agc act ctg att     1393
   Leu Leu Val Pro Thr Leu Phe Ser Phe Phe Ala Lys Ser Thr Leu Ile
                   290                 295                 300
   gac aaa tac gat tta tct aat tta cac gaa att gct tct ggg ggc gca     1441
   Asp Lys Tyr Asp Leu Ser Asn Leu His Glu Ile Ala Ser Gly Gly Ala
               305                 310                 315
   cct ctt tcg aaa gaa gtc ggg gaa gcg gtt gca aaa cgc ttc cat ctt     1489
   Pro Leu Ser Lys Glu Val Gly Glu Ala Val Ala Lys Arg Phe His Leu
           320                 325                 330
   cca ggg ata cga caa gga tat ggg ctc act gag act aca tca gct att     1537
   Pro Gly Ile Arg Gln Gly Tyr Gly Leu Thr Glu Thr Thr Ser Ala Ile
       335                 340                 345
   ctg att aca ccc gag ggg gat gat aaa ccg ggc gcg gtc ggt aaa gtt    1585
   Leu Ile Thr Pro Glu Gly Asp Asp Lys Pro Gly Ala Val Gly Lys Val
   350                 355                 360                 365
   gtt cca ttt ttt gaa gcg aag gtt gtg gat ctg gat acc ggg aaa acg    1633
   Val Pro Phe Phe Glu Ala Lys Val Val Asp Leu Asp Thr Gly Lys Thr
                   370                 375                 380
   ctg ggc gtt aat cag aga ggc gaa tta tgt gtc aga gga cct atg att    1681
   Leu Gly Val Asn Gln Arg Gly Glu Leu Cys Val Arg Gly Pro Met Ile
               385                 390                 395
   atg tcc ggt tat gta aac aat ccg gaa gcg acc aac gcc ttg att gac    1729
   Met Ser Gly Tyr Val Asn Asn Pro Glu Ala Thr Asn Ala Leu Ile Asp
           400                 405                 410
   aag gat gga tgg cta cat tct gga gac ata gct tac tgg gac gaa gac    1777
   Lys Asp Gly Trp Leu His Ser Gly Asp Ile Ala Tyr Trp Asp Glu Asp
       415                 420                 425
   gaa cac ttc ttc ata gtt gac cgc ttg aag tct tta att aaa tac aaa    1825
   Glu His Phe Phe Ile Val Asp Arg Leu Lys Ser Leu Ile Lys Tyr Lys
   430                 435                 440                 445
   gga tac cag gtg gcc ccc gct gaa ttg gaa tcg ata ttg tta caa cac    1873
   Gly Tyr Gln Val Ala Pro Ala Glu Leu Glu Ser Ile Leu Leu Gln His
                   450                 455                 460
   ccc aac atc ttc gac gcg ggc gtg gca ggt ctt ccc gac gat gac gcc    1921
   Pro Asn Ile Phe Asp Ala Gly Val Ala Gly Leu Pro Asp Asp Asp Ala
               465                 470                 475
   ggt gaa ctt ccc gcc gcc gtt gtt gtt ttg gag cac gga aag acg atg    1969
   Gly Glu Leu Pro Ala Ala Val Val Val Leu Glu His Gly Lys Thr Met
           480                 485                 490
   acg gaa aaa gag atc gtg gat tac gtc gcc agt caa gta aca acc gcg    2017
   Thr Glu Lys Glu Ile Val Asp Tyr Val Ala Ser Gln Val Thr Thr Ala
       495                 500                 505
   aaa aag ttg cgc gga gga gtt gtg ttt gtg gac gaa gta ccg aaa ggt    2065
   Lys Lys Leu Arg Gly Gly Val Val Phe Val Asp Glu Val Pro Lys Gly
   510                 515                 520                 525
   ctt acc gga aaa ctc gac gca aga aaa atc aga gag atc ctc ata aag    2113
   Leu Thr Gly Lys Leu Asp Ala Arg Lys Ile Arg Glu Ile Leu Ile Lys
                   530                 535                 540
   gcc aag aag ggc gga aag tcc aaa ttg aag gag tct act ctt cac ttg    2161
   Ala Lys Lys Gly Gly Lys Ser Lys Leu Lys Glu Ser Thr Leu His Leu
               545                 550                 555
   gtc ctc cgt ctc cgc ggt ggt atg cag atc ttc gta aag act ttg acc    2209
   Val Leu Arg Leu Arg Gly Gly Met Gln Ile Phe Val Lys Thr Leu Thr
           560                 565                 570
   gga aag acc atc act ctt gaa gtt gag agc tcc gac acc att gat aac    2257
   Gly Lys Thr Ile Thr Leu Glu Val Glu Ser Ser Asp Thr Ile Asp Asn
       575                 580                 585
   gtg aag gct aag atc cag gac aag gaa ggc att cct ccg gac cag cag    2305
   Val Lys Ala Lys Ile Gln Asp Lys Glu Gly Ile Pro Pro Asp Gln Gln
   590                 595                 600                 605
   cgt ctc atc ttc gct gga aag cag ctt gag gat gga cgt act ttg gcc    2353
   Arg Leu Ile Phe Ala Gly Lys Gln Leu Glu Asp Gly Arg Thr Leu Ala
                   610                 615                 620
   gac tac aac atc cag aag gag tct act ctt cac ttg gtc ctc cgt ctc 2401
   Asp Tyr Asn Ile Gln Lys Glu Ser Thr Leu His Leu Val Leu Arg Leu
   625 630 635
   cgc ggt ggt atg tta cgt cct gta gaa acc cca act cga gaa atc aaa 2449
   Arg Gly Gly Met Leu Arg Pro Val Glu Thr Pro Thr Arg Glu Ile Lys
   640 645 650
   aaa ctc gac ggc ctg tgg gca ttc agt ctg gat cgc gaa aac tgt gga 2497
   Lys Leu Asp Gly Leu Trp Ala Phe Ser Leu Asp Arg Glu Asn Cys Gly
   655 660 665
   att gat cag cgt tgg tgg gaa agc gcg tta caa gaa agc cgg gca att 2545
   Ile Asp Gln Arg Trp Trp Glu Ser Ala Leu Gln Glu Ser Arg Ala Ile
   670 675 680 685
   gct gtg cca ggc agt ttt aac gat cag ttc gcc gat gca gat att cgt 2593
   Ala Val Pro Gly Ser Phe Asn Asp Gln Phe Ala Asp Ala Asp Ile Arg
   690 695 700
   aat tat gcg ggc aac gtc tgg tat cag cgc gaa gtc ttt ata ccg aaa 2641
   Asn Tyr Ala Gly Asn Val Trp Tyr Gln Arg Glu Val Phe Ile Pro Lys
   705 710 715
   ggt tgg gca ggc cag cgt atc gtg ctg cgt ttc gat gcg gtc act cat 2689
   Gly Trp Ala Gly Gln Arg Ile Val Leu Arg Phe Asp Ala Val Thr His
   720 725 730
   tac ggc aaa gtg tgg gtc aat aat cag gaa gtg atg gag cat cag ggc 2737
   Tyr Gly Lys Val Trp Val Asn Asn Gln Glu Val Met Glu His Gln Gly
   735 740 745
   ggc tat acg cca ttt gaa gcc gat gtc acg ccg tat gtt att gcc ggg 2785
   Gly Tyr Thr Pro Phe Glu Ala Asp Val Thr Pro Tyr Val Ile Ala Gly
   750 755 760 765
   aaa agt gta cgt atc acc gtt tgt gtg aac aac gaa ctg aac tgg cag 2833
   Lys Ser Val Arg Ile Thr Val Cys Val Asn Asn Glu Leu Asn Trp Gln
   770 775 780
   act atc ccg ccg gga atg gtg att acc gac gaa aac ggc aag aaa aag 2881
   Thr Ile Pro Pro Gly Met Val Ile Thr Asp Glu Asn Gly Lys Lys Lys
   785 790 795
   cag tct tac ttc cat gat ttc ttt aac tat gcc gga atc cat cgc agc 2929
   Gln Ser Tyr Phe His Asp Phe Phe Asn Tyr Ala Gly Ile His Arg Ser
   800 805 810
   gta atg ctc tac acc acg ccg aac acc tgg gtg gac gat atc acc gtg 2977
   Val Met Leu Tyr Thr Thr Pro Asn Thr Trp Val Asp Asp Ile Thr Val
   815 820 825
   gtg acg cat gtc gcg caa gac tgt aac cac gcg tct gtt gac tgg cag 3025
   Val Thr His Val Ala Gln Asp Cys Asn His Ala Ser Val Asp Trp Gln
   830 835 840 845
   gtg gtg gcc aat ggt gat gtc agc gtt gaa ctg cgt gat gcg gat caa 3073
   Val Val Ala Asn Gly Asp Val Ser Val Glu Leu Arg Asp Ala Asp Gln
   850 855 860
   cag gtg gtt gca act gga caa ggc act agc ggg act ttg caa gtg gtg 3121
   Gln Val Val Ala Thr Gly Gln Gly Thr Ser Gly Thr Leu Gln Val Val
   865 870 875
   aat ccg cac ctc tgg caa ccg ggt gaa ggt tat ctc tat gaa ctg tgc 3169
   Asn Pro His Leu Trp Gln Pro Gly Glu Gly Tyr Leu Tyr Glu Leu Cys
   880 885 890
   gtc aca gcc aaa agc cag aca gag tgt gat atc tac ccg ctt cgc gtc    3217
   Val Thr Ala Lys Ser Gln Thr Glu Cys Asp Ile Tyr Pro Leu Arg Val
       895                 900                 905
   ggc atc cgg tca gtg gca gtg aag ggc gaa cag ttc ctg att aac cac    3265
   Gly Ile Arg Ser Val Ala Val Lys Gly Glu Gln Phe Leu Ile Asn His
   910                 915                 920                 925
   aaa ccg ttc tac ttt act ggc ttt ggt cgt cat gaa gat gcg gac tta    3313
   Lys Pro Phe Tyr Phe Thr Gly Phe Gly Arg His Glu Asp Ala Asp Leu
                   930                 935                 940
   cgt ggc aaa gga ttc gat aac gtg ctg atg gtg cac gac cac gca tta    3361
   Arg Gly Lys Gly Phe Asp Asn Val Leu Met Val His Asp His Ala Leu
               945                 950                 955
   atg gac tgg att ggg gcc aac tcc tac cgt acc tcg cat tac cct tac    3409
   Met Asp Trp Ile Gly Ala Asn Ser Tyr Arg Thr Ser His Tyr Pro Tyr
           960                 965                 970
   gct gaa gag atg ctc gac tgg gca gat gaa cat ggc atc gtg gtg att    3457
   Ala Glu Glu Met Leu Asp Trp Ala Asp Glu His Gly Ile Val Val Ile
       975                 980                 985
   gat gaa act gct gct gtc ggc ttt aac ctc tct tta ggc att ggt ttc    3505
   Asp Glu Thr Ala Ala Val Gly Phe Asn Leu Ser Leu Gly Ile Gly Phe
   990                 995                 1000                1005
   gaa gcg ggc aac aag ccg aaa gaa ctg tac agc gaa gag gca gtc aac    3553
   Glu Ala Gly Asn Lys Pro Lys Glu Leu Tyr Ser Glu Glu Ala Val Asn
                   1010                1015                1020
   ggg gaa act cag caa gcg cac tta cag gcg att aaa gag ctg ata gcg    3601
   Gly Glu Thr Gln Gln Ala His Leu Gln Ala Ile Lys Glu Leu Ile Ala
               1025                1030                1035
   cgt gac aaa aac cac cca agc gtg gtg atg tgg agt att gcc aac gaa    3649
   Arg Asp Lys Asn His Pro Ser Val Val Met Trp Ser Ile Ala Asn Glu
           1040                1045                1050
   ccg gat acc cgt ccg caa gtg cac ggg aat att tcg cca ctg gcg gaa    3697
   Pro Asp Thr Arg Pro Gln Val His Gly Asn Ile Ser Pro Leu Ala Glu
       1055                1060                1065
   gca acg cgt aaa ctc gac ccg acg cgt ccg atc acc tgc gtc aat gta    3745
   Ala Thr Arg Lys Leu Asp Pro Thr Arg Pro Ile Thr Cys Val Asn Val
   1070                1075                1080                1085
   atg ttc tgc gac gct cac acc gat acc atc agc gat ctc ttt gat gtg    3793
   Met Phe Cys Asp Ala His Thr Asp Thr Ile Ser Asp Leu Phe Asp Val
                   1090                1095                1100
   ctg tgc ctg aac cgt tat tac gga tgg tat gtc caa agc ggc gat ttg    3841
   Leu Cys Leu Asn Arg Tyr Tyr Gly Trp Tyr Val Gln Ser Gly Asp Leu
               1105                1110                1115
   gaa acg gca gag aag gta ctg gaa aaa gaa ctt ctg gcc tgg cag gag    3889
   Glu Thr Ala Glu Lys Val Leu Glu Lys Glu Leu Leu Ala Trp Gln Glu
           1120                1125                1130
   aaa ctg cat cag ccg att atc atc acc gaa tac ggc gtg gat acg tta    3937
   Lys Leu His Gln Pro Ile Ile Ile Thr Glu Tyr Gly Val Asp Thr Leu
       1135                1140                1145
   gcc ggg ctg cac tca atg tac acc gac atg tgg agt gaa gag tat cag    3985
   Ala Gly Leu His Ser Met Tyr Thr Asp Met Trp Ser Glu Glu Tyr Gln
   1150                1155                1160                1165
   tgt gca tgg ctg gat atg tat cac cgc gtc ttt gat cgc gtc agc gcc    4033
   Cys Ala Trp Leu Asp Met Tyr His Arg Val Phe Asp Arg Val Ser Ala
                   1170                1175                1180
   gtc gtc ggt gaa cag gta tgg aat ttc gcc gat ttt gcg acc tcg caa    4081
   Val Val Gly Glu Gln Val Trp Asn Phe Ala Asp Phe Ala Thr Ser Gln
               1185                1190                1195
   ggc ata ttg cgc gtt ggc ggt aac aag aaa ggg atc ttc act cgc gac    4129
   Gly Ile Leu Arg Val Gly Gly Asn Lys Lys Gly Ile Phe Thr Arg Asp
           1200                1205                1210
   cgc aaa ccg aag tcg gcg gct ttt ctg ctg caa aaa cgc tgg act ggc    4177
   Arg Lys Pro Lys Ser Ala Ala Phe Leu Leu Gln Lys Arg Trp Thr Gly
       1215                1220                1225
   atg aac ttc ggt gaa aaa ccg cag cag gga ggc aaa caa tgaatcaaca     4226
   Met Asn Phe Gly Glu Lys Pro Gln Gln Gly Gly Lys Gln
   1230                1235                1240
   actctcctgg cgcaccatcg tcggctacag cctcggtggg aattcctgca gggatccccg 4286
   gggatcgttc aaacatttgg caataaagtt tcttaagatt gaatcctgtt gccggtcttg 4346
   cgatgattat catataattt ctgttgaatt acgttaagca tgtaataatt aacatgtaat 4406
   gcatgacgtt atttatgaga tgggttttat gattagagtc ccgcaattat acatttaata 4466
   cgcgatagaa aacaaaatat agcgcgacta ggataaatta tcgcgcgcgg tgtcatctat 4526
   gttactagat ccgtcgac                                               4544
<210> 2
   <211> 1242
   <212> PRT
   <213> Artificial Sequence
   <223> Description of Artificial Sequence:Fusion Protein Expression Cassette
<400> 2
   Met Glu Asp Ala Lys Asn Ile Lys Lys Gly Pro Ala Pro Phe Tyr Pro
    1               5                  10                  15
   Leu Glu Asp Gly Thr Ala Gly Glu Gln Leu His Lys Ala Met Lys Arg
               20                  25                  30
   Tyr Ala Leu Val Pro Gly Thr Ile Ala Phe Thr Asp Ala His Ile Glu
           35                  40                  45
   Val Asn Ile Thr Tyr Ala Glu Tyr Phe Glu Met Ser Val Arg Leu Ala
       50                  55                  60
   Glu Ala Met Lys Arg Tyr Gly Leu Asn Thr Asn His Arg Ile Val Val
   65                  70                  75                  80
   Cys Ser Glu Asn Ser Leu Gln Phe Phe Met Pro Val Leu Gly Ala Leu
                   85                  90                  95
   Phe Ile Gly Val Ala Val Ala Pro Ala Asn Asp Ile Tyr Asn Glu Arg
               100                 105                 110
   Glu Leu Leu Asn Ser Met Asn Ile Ser Gln Pro Thr Val Val Phe Val
           115                 120                 125
   Ser Lys Lys Gly Leu Gln Lys Ile Leu Asn Val Gln Lys Lys Leu Pro
       130                 135                 140
   Ile Ile Gln Lys Ile Ile Ile Met Asp Ser Lys Thr Asp Tyr Gln Gly
   145                 150                 155                 160
   Phe Gln Ser Met Tyr Thr Phe Val Thr Ser His Leu Pro Pro Gly Phe
                   165                 170                 175
   Asn Glu Tyr Asp Phe Val Pro Glu Ser Phe Asp Arg Asp Lys Thr Ile
               180                 185                 190
   Ala Leu Ile Met Asn Ser Ser Gly Ser Thr Gly Leu Pro Lys Gly Val
           195                 200                 205
   Ala Leu Pro His Arg Thr Ala Cys Val Arg Phe Ser His Ala Arg Asp
       210                 215                 220
   Pro Ile Phe Gly Asn Gln Ile Ile Pro Asp Thr Ala Ile Leu Ser Val
   225                 230                 235                 240
   Val Pro Phe His His Gly Phe Gly Met Phe Thr Thr Leu Gly Tyr Leu
                   245                 250                 255
   Ile Cys Gly Phe Arg Val Val Leu Met Tyr Arg Phe Glu Glu Glu Leu
               260                 265                 270
   Phe Leu Arg Ser Leu Gln Asp Tyr Lys Ile Gln Ser Ala Leu Leu Val
           275                 280                 285
   Pro Thr Leu Phe Ser Phe Phe Ala Lys Ser Thr Leu Ile Asp Lys Tyr
       290                 295                 300
   Asp Leu Ser Asn Leu His Glu Ile Ala Ser Gly Gly Ala Pro Leu Ser
   305                 310                 315                 320
   Lys Glu Val Gly Glu Ala Val Ala Lys Arg Phe His Leu Pro Gly Ile
                   325                 330                 335
   Arg Gln Gly Tyr Gly Leu Thr Glu Thr Thr Ser Ala Ile Leu Ile Thr
               340                 345                 350
   Pro Glu Gly Asp Asp Lys Pro Gly Ala Val Gly Lys Val Val Pro Phe
           355                 360                 365
   Phe Glu Ala Lys Val Val Asp Leu Asp Thr Gly Lys Thr Leu Gly Val
       370                 375                 380
   Asn Gln Arg Gly Glu Leu Cys Val Arg Gly Pro Met Ile Met Ser Gly
   385                 390                 395                 400
   Tyr Val Asn Asn Pro Glu Ala Thr Asn Ala Leu Ile Asp Lys Asp Gly
                   405                 410                 415
   Trp Leu His Ser Gly Asp Ile Ala Tyr Trp Asp Glu Asp Glu His Phe
               420                 425                 430
   Phe Ile Val Asp Arg Leu Lys Ser Leu Ile Lys Tyr Lys Gly Tyr Gln
           435                 440                 445
   Val Ala Pro Ala Glu Leu Glu Ser Ile Leu Leu Gln His Pro Asn Ile
       450                 455                 460
   Phe Asp Ala Gly Val Ala Gly Leu Pro Asp Asp Asp Ala Gly Glu Leu
   465                 470                 475                 480
   Pro Ala Ala Val Val Val Leu Glu His Gly Lys Thr Met Thr Glu Lys
                   485                 490                 495
   Glu Ile Val Asp Tyr Val Ala Ser Gln Val Thr Thr Ala Lys Lys Leu
   500 505 510
   Arg Gly Gly Val Val Phe Val Asp Glu Val Pro Lys Gly Leu Thr Gly
   515 520 525
   Lys Leu Asp Ala Arg Lys Ile Arg Glu Ile Leu Ile Lys Ala Lys Lys
   530 535 540
   Gly Gly Lys Ser Lys Leu Lys Glu Ser Thr Leu His Leu Val Leu Arg
   545 550 555 560
   Leu Arg Gly Gly Met Gln Ile Phe Val Lys Thr Leu Thr Gly Lys Thr
   565 570 575
   Ile Thr Leu Glu Val Glu Ser Ser Asp Thr Ile Asp Asn Val Lys Ala
   580 585 590
   Lys Ile Gln Asp Lys Glu Gly Ile Pro Pro Asp Gln Gln Arg Leu Ile
   595 600 605
   Phe Ala Gly Lys Gln Leu Glu Asp Gly Arg Thr Leu Ala Asp Tyr Asn
   610 615 620
   Ile Gln Lys Glu Ser Thr Leu His Leu Val Leu Arg Leu Arg Gly Gly
   625 630 635 640
   Met Leu Arg Pro Val Glu Thr Pro Thr Arg Glu Ile Lys Lys Leu Asp
   645 650 655
   Gly Leu Trp Ala Phe Ser Leu Asp Arg Glu Asn Cys Gly Ile Asp Gln
   660 665 670
   Arg Trp Trp Glu Ser Ala Leu Gln Glu Ser Arg Ala Ile Ala Val Pro
   675 680 685
   Gly Ser Phe Asn Asp Gln Phe Ala Asp Ala Asp Ile Arg Asn Tyr Ala
   690 695 700
   Gly Asn Val Trp Tyr Gln Arg Glu Val Phe Ile Pro Lys Gly Trp Ala
   705 710 715 720
   Gly Gln Arg Ile Val Leu Arg Phe Asp Ala Val Thr His Tyr Gly Lys
   725 730 735
   Val Trp Val Asn Asn Gln Glu Val Met Glu His Gln Gly Gly Tyr Thr
   740 745 750
   Pro Phe Glu Ala Asp Val Thr Pro Tyr Val Ile Ala Gly Lys Ser Val
   755 760 765
   Arg Ile Thr Val Cys Val Asn Asn Glu Leu Asn Trp Gln Thr Ile Pro
   770 775 780
   Pro Gly Met Val Ile Thr Asp Glu Asn Gly Lys Lys Lys Gln Ser Tyr
   785 790 795 800
   Phe His Asp Phe Phe Asn Tyr Ala Gly Ile His Arg Ser Val Met Leu
   805 810 815
   Tyr Thr Thr Pro Asn Thr Trp Val Asp Asp Ile Thr Val Val Thr His
   820 825 830
   Val Ala Gln Asp Cys Asn His Ala Ser Val Asp Trp Gln Val Val Ala
   835 840 845
   Asn Gly Asp Val Ser Val Glu Leu Arg Asp Ala Asp Gln Gln Val Val
   850 855 860
   Ala Thr Gly Gln Gly Thr Ser Gly Thr Leu Gln Val Val Asn Pro His
   865 870 875 880
   Leu Trp Gln Pro Gly Glu Gly Tyr Leu Tyr Glu Leu Cys Val Thr Ala
   885 890 895
   Lys Ser Gln Thr Glu Cys Asp Ile Tyr Pro Leu Arg Val Gly Ile Arg
   900 905 910
   Ser Val Ala Val Lys Gly Glu Gln Phe Leu Ile Asn His Lys Pro Phe
   915 920 925
   Tyr Phe Thr Gly Phe Gly Arg His Glu Asp Ala Asp Leu Arg Gly Lys
   930 935 940
   Gly Phe Asp Asn Val Leu Met Val His Asp His Ala Leu Met Asp Trp
   945 950 955 960
   Ile Gly Ala Asn Ser Tyr Arg Thr Ser His Tyr Pro Tyr Ala Glu Glu
   965 970 975
   Met Leu Asp Trp Ala Asp Glu His Gly Ile Val Val Ile Asp Glu Thr
   980 985 990
   Ala Ala Val Gly Phe Asn Leu Ser Leu Gly Ile Gly Phe Glu Ala Gly
   995 1000 1005
   Asn Lys Pro Lys Glu Leu Tyr Ser Glu Glu Ala Val Asn Gly Glu Thr
   1010 1015 1020
   Gln Gln Ala His Leu Gln Ala Ile Lys Glu Leu Ile Ala Arg Asp Lys
   1025 1030 1035 1040
   Asn His Pro Ser Val Val Met Trp Ser Ile Ala Asn Glu Pro Asp Thr
   1045 1050 1055
   Arg Pro Gln Val His Gly Asn Ile Ser Pro Leu Ala Glu Ala Thr Arg
   1060 1065 1070
   Lys Leu Asp Pro Thr Arg Pro Ile Thr Cys Val Asn Val Met Phe Cys
   1075 1080 1085
   Asp Ala His Thr Asp Thr Ile Ser Asp Leu Phe Asp Val Leu Cys Leu
   1090 1095 1100
   Asn Arg Tyr Tyr Gly Trp Tyr Val Gln Ser Gly Asp Leu Glu Thr Ala
   1105 1110 1115 1120
   Glu Lys Val Leu Glu Lys Glu Leu Leu Ala Trp Gln Glu Lys Leu His
   1125 1130 1135
   Gln Pro Ile Ile Ile Thr Glu Tyr Gly Val Asp Thr Leu Ala Gly Leu
   1140 1145 1150
   His Ser Met Tyr Thr Asp Met Trp Ser Glu Glu Tyr Gln Cys Ala Trp
   1155 1160 1165
   Leu Asp Met Tyr His Arg Val Phe Asp Arg Val Ser Ala Val Val Gly
   1170 1175 1180
   Glu Gln Val Trp Asn Phe Ala Asp Phe Ala Thr Ser Gln Gly Ile Leu
   1185 1190 1195 1200
   Arg Val Gly Gly Asn Lys Lys Gly Ile Phe Thr Arg Asp Arg Lys Pro
   1205 1210 1215
   Lys Ser Ala Ala Phe Leu Leu Gln Lys Arg Trp Thr Gly Met Asn Phe
   1220 1225 1230
   Gly Glu Lys Pro Gln Gln Gly Gly Lys Gln
   1235 1240